# EUROPEAN PATENT SPECIFICATION

(11) **EP 2 619 253 B1**
(45) Date of publication and mention of the grant of the patent: **23.07.2014**
(21) Application number: 11757806.2
(22) Date of filing: 01.09.2011
(51) Int. Cl.: C08J 3/075, C08B 37/00, C08L 1/08, C08L 91/00, C08L 89/06, C08L 89/00, C08L 5/00, A61K 8/73, A61Q 19/00, C08L 5/06, C08L 5/08, C08L 5/12

(54) **HIGHLY CONCENTRATED, SPHERICAL BIOPOLYMER GEL PARTICLE SUSPENSIONS PREPARED BY HIPE-GELATION PROCESS**
ANHAND EINES HIPE-GELIERVERFAHRENS HERGESTELLTE SUSPENSIONEN AUS HOCHKONZENTRIERTEN KUGELFÖRMIGEN BIOPOLYMERGELPARTIKELN
SUSPENSIONS DE PARTICULES DE GEL BIOPOLYMÈRE SPHÉRIQUES HAUTEMENT CONCENTRÉES PRÉPARÉES PAR UN PROCÉDÉ DE GÉLIFICATION D'ÉMULSIONS À PHASE INTERNE CONCENTRÉE

(30) Priority: 24.09.2010 US 889657; 24.09.2010 US 889618
(43) Date of publication of application: 31.07.2013
(73) Proprietor: Unilever PLC, London, Greater London EC4Y 0DY (GB); Unilever N.V., 3013 AL Rotterdam (NL)
(72) Inventor: QUAN, Congling, Trumbull, Connecticut 06611 (US); MOADDEL, Teanoosh, Trumbull, Connecticut 06611 (US); AHTCHI-ALI, Badreddine, Trumbull, Connecticut 06611 (US)
(74) Representative: Fijnvandraat, Arnoldus
(86) International application number: PCT/EP2011/065141
(87) International publication number: WO 2012/038238

(56) References cited:
- WO-A1-2010/009989
- WO-A2-2010/097370
- US-A- 3 959 251

## Description

The present invention relates to novel suspensions comprising highly concentrated spherical biopolymer gel particles. The suspensions are prepared using a novel HIPE-Gelation process (where HIPE is the intermediate product prior to cooling to form the final suspensions) where water-in-oil high internal phase emulsions (HIPEs) comprising water soluble biopolymers (e.g. agarose) are formed at elevated temperatures and the HIPEs are subsequently cooled such that the water phase gels to form the novel, highly concentrated spherical biopolymer gel particle suspensions. The HIPE intermediates are formed at elevated temperature from the combination of (1) an aqueous phase comprising biopolymer in polar or water solvent solution (optionally further comprising water soluble actives); and (2) nonionic surfactant in oil solutions.

Through proper selection of surfactants/oils; biopolymer concentration (e.g., ratio of biopolymer to solvent); and mixing conditions, e.g., temperature and shear, it is possible to prepare a high concentration of spherical particles (in the suspension upon gellation) having the desired size range and excellent sensory characteristics. Moreover, the suspensions can be prepared on a commercial scale in a simple and efficient process where concentrated spherical particles are preferably prepared without the need of a homogenizer operating under pressure up to 20,000 psi (137.9 MPa) or even up to 45,000 psi (310.3 Mpa). The concentrated, spherical gel particle suspensions made according to this invention may be used "as is", or may be incorporated into aqueous or oil based personal care product to deliver a unique sensory feel.

High internal phase emulsions have been known for many years, and have found applications in areas such as food preparation, fuels, oil recovery and cosmetics. Typically, HIPEs are defined as a class of emulsions with a volume fraction of dispersed (internal) phase above 0.74. Examples include mayonnaise, an oil-in-water HIPE with greater than 75% oil droplets suspended in less than 25% external water phase; and Dove^{®} smooth and soft anti-frizz cream, a water-in-oil HIPE with 80% water droplets suspended in less than 20% continuous silicone phase.

High internal phase emulsions are also widely used as a template to create highly porous materials. For example, the internal phase is water and the external oil phase consists of polymerizable monomers. Upon polymerization and removal of the internal water phase a highly porous cellular structure is created. This is widely referred to as PolyHIPE.

In the cosmetic and personal care industry, particles have been widely used in products to provide unique sensory attributes. Biopolymer particles (e.g., agarose, carrageenan) have gained considerable ground, due to their unique properties, as well as to their environmental friendliness or biodegradability.

In a co-pending application U.S. Serial No. 12/392,646, entitled "Shear Gels and Compositions Comprising Shear Gel", filed February 25, 2009, the applicants disclose shear gel compositions which comprise biopolymer particles prepared in water or polar solvent. These shear gels are prepared by heating a biopolymer/solvent mixture and cooling, with shear, through the gellation temperature of the biopolymer. Particles produced upon cooling or gellation are irregular in shape (e.g., are not predominantly spherical), and have a diameter varying from about 1 to 200 micrometer, preferably 8 to 150 micrometer. The shearing apparatus comprises a homogenizer operating under a pressure up to 20,000 psi (pounds per square inch) (137.9 MPa), or even up to 45,000 psi (310.3 MPa).

S. Hjerter (Biochim. Biophys. Acta, 79 (1964) 393-398), discloses a method to prepare spherical agarose particles via suspension-gellation for application in chromatography. Hot agarose solution is poured into an organic liquid containing hydrophobic stabilizer followed by cooling under agitation. The suspension formed is not a concentrated gel particle suspension and the final suspension contains less than 40% agarose gel. Further, toxic, organic solvents (e.g., toluene) are used in preparation of the suspension and those solvents must be removed. More recently Q-Z Zhou et al (Journal of Colloid and Interface Science 311 (2007) 118-127) reported a preparation method of uniform-sized agarose beads prepared using a microporous membrane emulsification technique. The technique comprises pressing hot agarose solution through uniform-sized pores of the membrane into the oil phase.

Although both methods produced suspensions comprising spherical agarose beads, they have disadvantages in applications in personal care industry and production scale-up. S. Hjerter's method creates a suspension containing less than 40% agarose gel (e.g., is not concentrated as required by suspension of our product). Further, toxic organic solvents must be removed in order to be suitable in personal care industry; filtering/washing of beads increases the process complexity, generates waste and increases cost. Q-Z Zhou's method has issues in scale-up as processes involving microporous membranes generally do. Further, only dilute emulsions (i.e., these are not concentrated suspensions) were reported via the microporous membrane emulsification technique.

The present invention is directed to novel highly concentrated, spherical gel particle suspensions (as well as to a process for making the suspensions) wherein a high internal phase emulsion (HIPE) is used as a template or intermediate to create the final concentrated, spherical biopolymer gel particle suspension (i.e., by passing through an intermediate HIPE phase in processing, a novel suspension is formed upon gellation). The final suspension prepared may be used in aqueous or oil based personal care products, or alternatively, gel particle suspensions formed by the process of this invention can be used as is. In addition to the novel product, the process for forming the gel particle suspensions (via the HIPE-gelation process) is itself novel. The process comprises 1) dissolving biopolymer into water or polar solvent (and optional water soluble actives) at elevated temperature to form highly concentrated internal phase of the HIPE; 2) forming an external oil phase by mixing nonionic surfactant and oils; 3) gradually adding biopolymer solution into the oil phase under moderate agitation (e.g., high pressure homogenization is preferably avoided) to form the HIPE intermediate; and 4) cooling the mixture to a temperature below the biopolymer gelation temperature, to form a suspension comprising the concentrated, spherical biopolymer particles of desired size and elasticity.

Desired elasticity of particles in the suspension can be manipulated by varying biopolymer concentration used to form the internal aqueous phase forming HIPE intermediate (e.g., using 0.01 to 15%, preferably, 1 to 10% by wt. biopolymer as starting material relative to the aqueous phase). Desired size of particles can be manipulated by choice of oil and or surfactant and or shear through cooling. By this process a suspension can be produced, upon cooling, whereby sensory gel particles can make up 60 to 99% by wt. of the final suspension product. The level of surfactant (having HLB <15, preferably <10, more preferably <7) used in preparation of the HIPE intermediate can be as low as 0.01% by wt. The process may be readily carried out in a general purpose mixer known to those skilled in the art. The particle suspension prepared in this way may be incorporated into an aqueous or oil based personal care product without any destabilizing effects.

The above described process is referred to as HIPE-Gelation process and does not require the use of (and preferably avoids use of) high shear devices (e.g. Silverson Rotor-Stator mixer) or high pressure homogenizer, resulting in significant energy saving and savings in capital investment. Furthermore, because the final gel suspension may contain less than 10% by volume nonionic surfactant and oils (used in formation of the HIPE prior to cooling to form suspension), a high yield of sensory particles (concentrated suspension) is produced. Furthermore, the nonionic surfactant and oils used in forming the HIPE intermediate may be chosen from a range of widely used surfactants and oils in the personal care industry. The suspensions formed from the process are novel in that they comprise highly concentrated, spherical, gel particle suspensions (with some nonionic surfactant and some oil into which particles are suspended). Any composition comprising the novel suspensions prepared by said novel process are, of course, themselves novel.

In one embodiment, the present invention is directed to highly concentrated, spherical biopolymer gel particle suspensions comprising:
1) 60 to 99 wt%, preferably greater than 74 to 95 wt% of the final suspension of spherical biopolymer gel particles (formed after cooling the HIPE intermediate product); the biopolymer gel particle suspension is produced via cooling (gellation) of a water-in-oil high internal phase emulsion (HIPE gellation process), wherein a water or polar solvent solution comprising biopolymer (existing as the internal aqueous phase of the HIPE) is combined with external oil phase to form HIPE and is then cooled to form the suspension. The ratio of biopolymer to water or polar solvent in forming the aqueous phase of the HIPE is 0.01/99.99% to 15/85% by wt, preferably 2/98% to 5/95% by wt. The biopolymer gel particles are spherical upon production of the suspension; and the average diameter of the particles in the suspension is 1-50 micrometer, preferably 5 to 40 micrometer; and
2) 1 to 40% by wt., preferably 1 to 20%, more preferably 1 to 10% by wt. of the final suspension of oils and surfactants used in formation of the HIPE intermediate which in turn is used to form the suspension and which are found in the HIPE (as well as in final suspension) in the following amounts:
   (a) 0.1 to 30 wt%, preferably 1 to 9 wt% of the HIPE intermediate of an oil or oil mixture which functions as the suspending medium for the above mentioned biopolymer particles in (1) upon formation of the suspension;
   (b) 0.01 to 10 wt%, preferably 0.1 to 2% wt. of the HIPE intermediate of a surfactant or surfactants which are dissolved or dispersed in the above mentioned oil or oil mixture in a),
wherein said surfactant preferably comprises a nonionic surfactant and has a hydrophilic-lipophilic balance (HLB) less than 15, preferably less than 10, more preferably less than 7;

The size of the spherical gel particles, and viscosity of the suspensions formed upon gellation are affected by the particular biopolymer molecule used; the concentration of the biopolymer relative to the total amount of water (e.g., 0.01-15% biopolymer as starting material when forming internal aqueous phase); the particular surfactant and oils used; and the temperature at which the HIPE intermediate is formed. These conditions can thus be used to control the physical properties (size and hardness of particles) of the biopolymer gel particles in the final suspension which in turn will impact the sensory properties delivered from these particles when used "as is" or when added to a product.

The dispersions are prepared by a HIPE-gelation process (second embodiment) which is used to prepare the highly concentrated spherical, biopolymer gel particle suspensions. This process comprises:
(a) forming an aqueous phase by dissolving 0.01 to 15% by wt., preferably 1 to 10% by wt. biopolymer into water and/or polar solvent, as well as optional water-soluble active (ratio of biopolymer to water and/or polar solvent is 0.1/99.9 to 15/85 by wt.), in a properly sized vessel; and heating said solution to a temperature, typically 60° to 100°C, preferably 70° to 90°C, which is above the gellation temperature of the biopolymer to produce a homogeneous mixture deplete of non-swollen biopolymer particulate (e.g., all biopolymer is dissolved in the aqueous phase);
(b) dissolving or dispersing a surfactant or surfactants into an oil or oil mixture in a separate properly sized vessel , and heating the said solution or dispersion to a temperature above the gellation temperature of the biopolymer, typically 60 to 100°C, preferably 70° to 90°C;
   wherein said surfactant comprises preferably nonionic surfactant(s) having an HLB less than 15, preferably less than 10, more preferably less than 7;
(c) dispersing biopolymer solution of (a) into the said oil solution or dispersion of (b) preferably with agitation at a temperature, typically 60° to 100°C, preferably 70° to 90°C, which is above the gellation temperature of the biopolymer wherein said biopolymer solution is dispersed into the oil mixture containing (1) surfactant (0.01 to 10%, preferably 0.1 to 2% wt. of the overall HIPE intermediate formulation; this is the same amount that will be found in suspension upon cooling); and (2) oils (0.1 to 30%, preferably 1 to 9% by wt of the overall HIPE intermediate formulation; same amount that will be found in suspension upon cooling) at a temperature (preferably 70° to 90°C) which is above the gelation temperature of the biopolymer, all done with moderate agitation; a water-in-oil high internal phase emulsion (HIPE) is formed with the aqueous biopolymer phase.as internal phase, in the form of small spherical droplets of 1 to 50 microns suspended in oils which is the continuous phase (this is the HIPE intermediate product); and
(d) cooling (gellation step in the HIPE gellation process) said HIPE containing biopolymer solution as internal aqueous phase; the viscosity of biopolymer solution within the said small spherical droplets of 1 to 50 micrometer increases as a result of hydrogen bonding between biopolymer molecules. When cooling to below biopolymer gellation temperature, typically 25° to 50°C, preferably 30° to 40°C, the biopolymer solution droplets gel to form gel particles, wherein said gel particles have size ranging from 1-50 micrometer in diameter and wherein the particles are spherical

The aqueous biopolymer phase (e.g., biopolymer in water or in polar solvent) may be as high as 99% by wt. (60 to 99% by wt.) of the final suspension after gellation (same amount as found in intermediate HIPE before gellation) and surfactants and oil may together be 40% or less by weight of the suspension. Preferably, oil phase wt. % in final suspension is 20% or less, more preferably 1 to 10%. As noted, gel particles will form at the gellation temperature of the biopolymer. For example, when agarose is used, the water droplets harden to gel particles at temperatures less than 35-40°C.

The size of the gel particles, and viscosity of the final suspensions are affected by the particular biopolymer molecule used; concentration of the biopolymer (e.g., 0.01 to 15% biopolymer dissolved in the water and/or solvent); the particular surfactant and oil used; and the temperature at which the HIPE is formed. These conditions can thus be used to control the physical properties (size and hardness of particles) of the biopolymer gel particles which in turn will impact the sensory properties delivered from these particles.

In another embodiment, the subject invention relates to a method to control sensory properties of biopolymer gels by controlling the size and hardness of gel particle suspensions formed. Desired size and texture of gel particles can be achieved by proper choice of (a) biopolymer; (b) concentration of biopolymer (0.01-15%, preferably 1-10% biopolymer in water and/or polar solvent plus optional water soluble colorants or skin beneficial actives); (c) surfactant type and concentration; and (d) type of oil.

Because the gel particle suspensions formed can be used as final product (e.g., to sell directly) or can be further integrated into a base to form final creams or multiple emulsions, for example, the manipulation of the gel particle suspension's physical property can be used to control sensory properties of the final product in which it is incorporated. Thus, the biopolymer concentration, choice of oils and/or surfactants, and the temperature at which the HIPE is formed may also be used to manipulate sensory properties of the final product.

In another embodiment, the invention may comprise topical compositions containing the gel particle suspension.

Finally, it should be noted that the gel particle suspensions of the invention may also be used to encapsulate water soluble actives (e.g., glycerine or hydroxypropyltri (C₁-C₃ alkyl) ammonium salts may be encapsulated within the gel particle) which can be released from the gel particles as moisturizing agents. The active can be incorporated when the particle suspensions are sold as produced; or, as noted above, the gel particle suspensions (with active encapsulated in the gel particles) may be introduced into a topical composition, one of the embodiments of the invention.

These and other aspects, features and advantages will become apparent to those of ordinary skill in the art from a reading of the following detailed description and the appended claims. For the avoidance of doubt, any feature of one aspect of the present invention may be utilized in any other aspect of the invention. It is noted that the examples given in the description below are intended to clarify the invention and are not intended to limit the invention to those examples per se.

Similarly, all percentages are weight/weight percentages of the total composition unless otherwise indicated. Numerical ranges expressed in the format "from x to y" are understood to include x and y. When for a specific feature multiple preferred ranges are described in the format "from x to y" it is understood that all ranges combining the different endpoints are also contemplated; and "x to y" are understood to subsume all values in this range. Where the term "comprising" is used in the specification or clams, it is not intended to exclude any terms, steps or features not specifically recited. All temperatures are in degrees Celsius (°C) unless specific otherwise. All measurements are in SI units unless specified otherwise.

The present invention relates to a novel highly concentrated (60 to 99% of final suspension), spherical biopolymer gel particle suspensions. The gel particles comprise a high volume fraction of the suspension. The particles themselves are formed by a combination of biopolymer and water or polar solvent which are formed initially in a heated solution (as aqueous internal phase) before combining with separate oil/surfactant phase to form a HIPE intermediate, and subsequently cooling to form gel suspension. It should be noted the biopolymer may also be encapsulating water soluble actives.

Unexpectedly, the applicants have found that, with proper selection of biopolymers (e.g., type and/or concentration), surfactants and oil; and with proper processing conditions, it is possible to produce these highly concentrated, spherical biopolymer gel particle suspensions. As indicated, this is done through a water-in-oil high internal phase emulsion (HIPE)-gellation process, which process is claimed in a second embodiment. The suspensions comprise highly concentrated, biopolymer gel particles which are spherical and with distribution of gel particles which range from 1-50 micrometer in diameter.

In terms of shape, uniformity of the gel, and ease of processing, the suspension differs from shear gels and processes of previous work applicant have done in recent filings (U.S. Serial No. 12/392,646, entitled "Shear Gels and Compositions Comprising Shear Gel", filed February 25, 2009). The biopolymer gels of '646 are irregular in shape compared to spherical in shape like the particles of this invention. Additionally, the polymer gels are not uniform and have polymer poor and polymer rich regions. The biopolymer gels of comparable particle size of '646 were not prepared using the emulsification process used in the subject invention. Further, particles of '646 were obtained with high pressure homogenizing, while the HIPE-gellation process of the present invention obtains concentrated, spherical particles even without such high pressure homogenization and associated use of high shear devices.

Thus, the suspensions made by the process of the present invention comprise highly concentrated spherical gel particle suspension (produced upon cooling of a heated HIPE solution) comprising:
1) 60% to 99% by wt. , preferably >74% to 95% by wt. of the final suspension of spherical biopolymer gel particles; and
2) 1 to 40%, preferably 1-20%, more preferably 1-10% by wt. of the final suspension of oils and surfactants wherein amounts of oils and surfactants used in the formation of the HIPE (prior to cooling to form the suspension) is:
   (a) 0.1 to 30%, preferably 1 to 9% by wt. of an oil or oil blends; and
   (b) 0.01 to 10% by wt., preferably 0.1 to 2% by wt. of a nonionic surfactant or surfactants blends.

The suspension is prepared via a HIPE intermediate. The HIPE is typically prepared by gradually dispersing an internal hot aqueous phase comprising biopolymer dispersed into water and/or polar solvent (and optional water soluble active) into a hot external phase comprising surfactant(s) dispersed into an oil or oil mixture. Dispersion of internal phase into external phase is done with moderate agitation in a general purpose mixer known to those skilled in the art (but preferably not under high pressure homogenization) to form the HIPE. Upon cooling to below the gellation temperature of the biopolymer, the droplets of the HIPE composition harden into a suspension comprising gel particles. The gel particles are of diameter 1 to 50, preferably 5 to 40 micrometer.

In another embodiment, the elasticity/hardness and the size of the biopolymer gel suspension can be controlled by controlling concentration of biopolymer (during preparation of aqueous phase), as well as choice of surfactant and oil (used during preparation of oil phase). In other words, these parameters can be used to control sensory properties of the concentrated, spherical, biopolymer gel particle suspension formed when the intermediate high temperature HIPE product is cooled; or to control sensory properties of the topical compositions in which the particle suspension is used.

Biopolymer suitable for use in this invention may be chosen from the group consisting of polysaccharides, proteins and mixtures thereof such as those disclosed in co-pending U.S. Application 12/392,646.

The biopolymer used as starting reagents are macromolecules suitable for swelling with water, polar solvent or both and may be synthetically made, but are normally produced by living organisms. Those pure biopolymer can be, for example, grain-like, powdery, and crystalline.

Preferably, the biopolymer can be chosen, for example, from carrageenan, furcellaran, pectin, alginate, agar, agarose, gellan, glucomannan (e.g., Konjac), galactomannan (e.g., locust bean gum, guar), xanthan, modified cellulose, glucan (e.g., starches, curdlan), gelatin, whey protein or mixtures thereof. More preferably, the biopolymer used is agar, agarose, carrageenan, or a mixture thereof. In a most preferred embodiment, the biopolymer used is agarose.

The biopolymers suitable for use in this invention are made commercially available from suppliers like FMC Corporation; National Starch and Chemical Co.; Cyber Colloids Ltd., as well as Hispangar, S.A. Additional descriptions of the types of biopolymers that may be used in this invention may be found in Food Gels, Chapter 1, edited by Peter Harris, Elsevier, 1990 and U.S. Patent Nos. 6,673,371 and 5,738,897.

The biopolymer can optionally be used in combination with a synthetic thickener. Illustrative thickeners which may be suitably used include alkylated polyvinylpyrrolidones like butylated polyvinyl pyrrolidone sold under the name Ganex® line by ISP Corporation, terephthalate polyesters like polypropylene terephthalate and ammonium acryloyldimethyltaurate/VP Copolymer, both sold under Aristoflex® line by Clariant A.G.; and mono alkyl esters of poly(methyl vinyl/ether maleic acid) sodium salt, like that included in the EZ Sperse® line made available by ISP Corporation, as well as (3-dimethylaminopropyl)-methacrylamide/3-methacryloylamidopropyl)-lauryl-dimethyl-ammonium chloride like that included in the Styleze® line made available by ISP Corporation.

Other thickeners suitable for use include those generally classified as acrylic acid/ethyl acrylate copolymers and carboxyvinyl polymers made available by the B.F. Goodrich Company under the Carbopol name. Such thickeners consist essentially of colloidally water-soluble poly-alkenyl polyether cross-linked polymer of acrylic acid crosslinked with a crosslinking agent like polyallyl sucrose or polyallyl pentaerythritol. These thickeners include, for example, Carbopol 934, 940, 950, 951, 980 and 981.

Other examples of suitable synthetic thickeners for use herein include those sold under the name Carbopol Ultrez 10, Carbopol Ultrez 21, Carbopol ETD2020, Carbopol 1342, Carbopol 1382, and Pemulen TR-1 (CTFA designation: Acrylates/10-30 Alkyl Acrylate Cross-polymer). Still other examples of suitable thickeners include those made available by Seppic under the names Sepigel 305 and Sepiplus. If desired, combinations of synthetic thickeners may be employed whereby those classified as acrylate-derived and/or terephthalate polyesters are generally preferred.

Typically, the concentration of the biopolymer relative to the amount of water or polar solvents in the formulation is about 0.01 to about 15%, preferably 0.1 to about 10%, most preferably about 0.2 to about 7% by wt. biopolymer including all ranges subsumed therein. When synthetic polymer is desired, the same typically makes up from about 0.001 to about 6%, and preferably, from about 0.01 to about 4.0%, and most preferably, from about 0.015 to about 2.5% by weight synthetic polymer and including all ranges subsumed therein.

The exact concentration of biopolymer is important for controlling the elasticity (hardness) of the biopolymer gel finally formed when the HIPE (in turn formed from combination of aqueous biopolymer phase and surfactant/oil solution) is cooled. That is by increasing the levels from less than 0.1% biopolymer to levels as high as 10-15% (relative to water/polar solvent) we can form biopolymer gel particles in the gel particle suspension which range in elasticity from less than 10² Pa to greater than 10⁴ Pa.

The solvent with which the biopolymer is combined may be water or a polar, hydrophilic solvent. Illustrative yet non-limiting examples of the type of polar solvent that may be used (with or without water) in this invention are sorbitol, hydroxypropyl sorbitol, glycerine, ethoxylated glycerol, propolylated glycerol, polyalkylene glycols like polyethylene glycol and polypropylene glycol, diethylene glycol, dipropylene glycol, triethylene glycol, 2-ethoxyethanol, hexylene glycol, butylene glycol, hexamatriol, or mixtures thereof.

It should be noted that the gel particles in the suspension can also be used to encapsulate water soluble colorants and skin benefit actives such as those selected from the group consisting of glycolic acid, amino acids, glycerine, hydroxypropyl tri (C₁ to C₃ alkyl) ammonium salts or mixtures thereof. When released from the particles for use (either if particle suspension is sold as a stand alone product, or incorporated into topical compositions), the particles can be used as moisturizers or other appropriate function.

As indicated, the solvent and biopolymer and optionally other thickeners, emollients to be included in the biopolymer particle gel are combined at elevated temperature (i.e., at temperature above gellation temperature of biopolymer), and dispersed into the oil phase to form the HIPE, and are cooled with moderate agitation using standard mixing equipment known to those skilled in the art to form a gel particle suspension.

When cooled below the gellation temperature, the biopolymer gel particles formed comprise 60 to 99%, preferably >74 to 95%, more preferably 80 to 95% by wt. of the final gel particle suspension.

The surfactants used in the formation of the HIPE (i.e., when biopolymer solution is combined with the surfactant/oil solution) are preferably low HLB nonionic emulsifiers but could contain low levels of other type of surfactants such as anionic, amphoteric, zwitterionic, cationic or mixtures thereof.

Preferably, the surfactant is a nonionic surfactant having a hydrophilic-lipophilic balance (HLB) of less than 15, preferably 10 or less, more preferably 7 or less. Typical examples of such low HLB surfactants include linear or branched or cross-linked dimethicone polymers modified with polyether and/or alkyl chain (e.g., Shin Etsu KF series with HLB<7, KSG 200 series, KSG 300 series, KSG 700 series, KSG 800 series, Abil em 90, 97); esters of sorbitan with fatty acids (e.g. Span20-80 series); cremophor A6 (ceteareth-6 and steary alcohol), A25 (ceteareth-25) and GS 32(polyglyceryl-3 distearate) from BASF, sucrose esters (eg. sucrose stearate S-170, -270,-370 -570 from Mitsubishi-Kagaku Foods Corporation), monoglyceride based surfactants.

Particularly, preferred surfactants include polyether modified cross-linked silicone polymers (e.g., PEG-15/lauryl dimethicone cross-polymer such as KSG-310^{®} from Shin Etsu); polyalkylene glycol derivatives of dimethicone (e.g., cetyl polyethylene glycol/polypropylene glycol-10/ 1 dimethicone, such as Abil EM90^{®}; PEG-10 dimethicone, such as KF^{®}-6017 from Shin-Etsu or lauryl PEG-9 polydimethylsiloxyethyl dimethicone, such as KF^{®}-6038 from Shin-Etsu); esters of sorbitan (e.g., sorbitan monooleate, such as Span^{®} 80 from Croda); and mixtures thereof.

Typically, surfactant comprises 0.01 to 10%, preferably 0.05 to 3%, more preferably 0.1-2% by wt. of the HIPE composition (referred to as suspension upon cooling) including all ranges subsumed therein.

The surfactant(s) are typically dissolved or dispersed in oil or oil blends. A wide range of oils can be used, including mineral oil, organic oil or silicone oils. Finally, oils which are widely used in the cosmetic industry may be used.

Preferably, the oil is selected from the group consisting of mineral oils (e.g., Pionier^{®} 6501, Lilac^{®} 100); silicone oils (e.g., DC200/50 cts. from Dow Corning); triglyceride oils (e.g., caprylic/capryl triglycerides); C₈-C₂₄ chain length ester derivatives of C₁-C₁₀ carbons (e.g., isopropyl myristate); and mixtures thereof. The oil or oil mixture (which function as suspension medium for biopolymers particles) comprises 0.1 to 30%, preferably 1 to 9% by wt. of the HIPE intermediate before the HIPE is cooled to form suspension. These are the same percentages of oil or oil mixture in the suspension when cooled.

It has been noted that certain specific combinations of oil and surfactant should be avoided in order to prepare stable HIPE. These include combination of Abil EM90^{®} and silicone oil wherein silicone oil is the only other oil present in the oil phase; or combination of Abil EM90^{®} and light mineral oil (e.g lilac 100). Surprisingly, Abil EM90^{®} functions well when mineral oil and silicone are combined together; or when heavier mineral oils are used.

Together the surfactant(s) and oil comprise the external phase of the HIPE (particle gel suspension upon cooling) and may comprise 1 to 40%, preferably 1 to 10% by wt. of the final suspension (upon cooling).

Upon cooling of the HIPE, the gelled biopolymer solution in the internal phase will comprise particles which are spherical in shape and the particles will have a diameter of between 1-50µm, preferably 5-40µm, more preferably 10-25µm.

In another aspect of the invention, the invention comprises a HIPE gelation process for making the novel gel particle suspension of the subject invention.

The process of this aspect of the invention involves forming a solution of biopolymer as defined above and water and/or polar solvent, preferably with mixing wherein the biopolymer-solvent mixture is heated to a temperature above the gellation temperature of the biopolymer and preferably to a temperature that is above the gellation temperature of the resulting biopolymer. Preferably, the mixture is heated to a temperature from about 60°C to about 100°C, and most preferably, to a temperature from about 70°C to about 90°C, including all ranges subsumed therein. Heating occurs until a homogeneous mixture is prepared. Separately, a solution of surfactant or surfactants and oil, both as described above, is prepared and the biopolymer solution and surfactant solution are then combined to form a HIPE using standard mixing equipment (e.g., preferably, with no high pressure homogenization).

The HIPE solution is then cooled through the gellation temperature of the biopolymer to form the concentrated spherical gel particle suspension of the subject invention. The HIPE may be stirred while cooling using standard mixing equipment.

It is a key aspect of the invention, as noted, that the gel suspension may be formed while avoiding high-pressure homogenization; and while obtaining high concentration of biopolymer gel particles (e.g., from the internal phase of the HIPE) which are in a spherical shape and have a size ranging from 1-50µm.

In another aspect of the invention, the invention comprises a method of manipulating the hardness of the biopolymer gel particles in the gel particle suspension and, therefore, influencing sensory properties.

This can be done in a number of ways which include (1) selection of surfactant and/or oil into which the biopolymer solution will be dispersed when forming the HIPE (prior to gellation); (2) controlling the concentration of the biopolymer itself in biopolymer/solvent solution and (3) control of agitation rate during HIPE formation (again when biopolymer solution and oil/surfactant solutions are combined). Specifically, this can control the hardness and size of the spherical particles (upon cooling of HIPE) in a way which allows the applicants to modulate exactly what sensory feeling they wish to provide (based, for example, on evaluation of consumer sensory panels).

It is noted that the resulting highly concentrated, spherical, gel particle suspensions can be used or sold as final products with sensory profiles as determined by controlling the factors noted; or they can be made and sold as intermediate products to be used in topical compositions. The properties of the intermediate products can of course also be controlled depending on what effect is desired to be imparted on this final composition.

In another aspect of the invention, the invention relates to the use of the gel particle suspension in topical compositions.

Topical compositions of the present invention can, for example, be in the form of foam, liquid, lotion, cream, serum, gel, soap bar, cleansing product (e.g., body wash, facial wash or shampoo and conditioner) or toner, or applied via a face mask or patch. The topical composition of this invention is, preferably, a leave-on composition. Skin to which topical compositions are applied is meant to include skin on the face, neck, chest, back, arms, hands, buttocks, legs and scalp.

If used as part of a topical composition, the gel particle suspensions of the present invention can make up from about 1 to about 99%, and preferably, from about 3 to about 85%, and most preferably, from about 8 to about 60% by weight of the topical composition, based on total weight of the topical composition and including all ranges subsumed therein.

It should be known, however, that commercially acceptable and conventional vehicles may be used, acting as diluents and/or dispersants for the topical compositions of this invention, along with the gel particle suspensions (GPS). Therefore, the cosmetically acceptable vehicle suitable for use in this invention may be aqueous-based, anhydrous, oil-based or an emulsion, including a multiple emulsion. If the use of water is desired, water typically makes up the balance of the topical composition. Silicone elastomers are typically not preferred in this invention since the biopolymers found in the GPS described herein are, surprisingly, excellent silicone elastomer mimetics. Optionally, however, silicone elastomers may be used along with the GPS.

In addition to water, organic solvents may be optionally included to act along with the GPS within the topical compositions of the present invention. Illustrative and non-limiting examples of the types of organic solvents suitable for use in the present invention include alkanols like ethyl and isopropyl alcohol, or mixtures thereof.

Other optional additives suitable for use along with the HIPEs of this invention include ester oils like isopropyl myristate, cetyl myristate, 2-octyldodecyl myristate, avocado oil, almond oil, olive oil, sunflower seed oil, neopentylglycol dicaprate, or mixtures thereof. Typically, such ester oils are used at an amount to yield a stable, and most preferably, water-in-oil emulsion when such an emulsion is desired. Other oils suitable for use include those generally classified as hydrocarbons, including those known as waxes.

Emollients may also be used, if desired, within the topical composition of the present invention. Alcohols like 1-hexadecanol (i.e., cetyl alcohol) are often desired as are the emollients generally classified as silicone oils and synthetic esters. Silicone oils suitable for use include cyclic or linear polydimethylsiloxanes containing from 3 to 9, preferably from 4 to 5, silicon atoms. Non-volatile silicone oils useful as an emollient material in the inventive topical composition described herein include polyalkyl siloxanes, polyalkylaryl siloxanes and polyether siloxane copolymers. The essentially non-volatile polyalkyl siloxanes useful herein include, for example, polydimethylsiloxanes.

The ester emollients that may optionally be used are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, stearyl stearate and arachidyl behenate.
(5) Sterol esters, of which cholesterol fatty acid esters are examples.

Emollients, when used, typically make up from about 0.1 to about 50% by weight of the topical composition, including all ranges subsumed therein.

Fatty acids having from 10 to 30 carbon atoms may also be included within the composition of the present invention. Illustrative examples of such fatty acids include pelargonic, lauric, myristic, palmitic, stearic, isostearic, oleic, linoleic, arachidic, behenic or erucic acid, and mixtures thereof. Compounds that are believed to enhance skin penetration, like dimethyl sulfoxide, may also be used.

The polar solvents described herein may also be added as humectants in the desired topical composition of this invention. Therefore, such polar solvents may be used to make GPS, only as a humectant as an additive to the topical composition, or both. In an especially preferred embodiment, the topical composition of this invention has less than about 50% by weight polar solvent, and preferably, from about 0.001 to about 25% by weight polar solvent based on total weight of the topical composition and including all ranges subsumed therein.

Collectively, water, biopolymer gels, silicones, esters, fatty acids and/or humectants will be present in amounts from 1 to 99.9%, preferably from 80 to 99% by weight.

Surfactants may also be present in the topical compositions of the present invention. Total concentration of the surfactant will range from about 0 to about 40%, and preferably from about 0 to about 20%, optimally from about 0.001 to about 5% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; mono- and di- fatty acid esters of ethylene glycol; fatty acid monoglyceride; sorbitan, mono- and di- C₈-C₂₀ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C₈-C₂₀ acyl isethionates, acyl glutamates, C₈-C₂₀ alkyl ether phosphates and combinations thereof. In an especially preferred embodiment, the surfactant employed is nonionic, and especially, polyoxyethylene sorbitan monopalmitate sold as Tween 40 by ICI Americas, Inc.

Perfumes may be used in the topical composition of this invention. Illustrative non-limiting examples of the types of perfumes that may be used include those comprising terpenes and terpene derivatives like those described in Bauer, K., et al., Common Fragrance and Flavor Materials, VCH Publishers (1990).

Illustrative yet non-limiting examples of the types of fragrances that may be used in this invention include myrcene, dihydromyrenol, citral, tagetone, cis-geranic acid, citronellic acid, or mixtures thereof.

Preferably, the amount of fragrance employed in the topical composition of this invention is in the range from about 0.0% to about 10%, more preferably, about 0.00001% to about 5 wt %, most preferably, about 0.0001% to about 2%.

Various types of optional ingredients/additives may be used in the topical compositions of the present invention. Although not limited to this category, general examples include talcs and silicas, as well as alpha-hydroxy acids, beta-hydroxy acids, zinc salts, and sunscreens.

Beta-hydroxy acids include salicylic acid, for example. Zinc pyrithione is an example of the zinc salts useful in the topical composition of the present invention.

Sunscreens include those materials commonly employed to block ultraviolet light. Illustrative compounds are the derivatives of PABA, cinnamate and salicylate. For example, avobenzophenone (Parsol 1789^{®}) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the compositions can vary depending upon the degree of protection desired from the sun's UV radiation. Additives that reflect or scatter the suns rays may also be employed. These additives include oxides like zinc oxide and titanium dioxide.

Many topical compositions, especially those containing water, should be protected against the growth of potentially harmful microorganisms. Anti-microbial compounds, such as triclosan, and preservatives are, therefore, typically necessary. Suitable preservatives include alkyl esters of p-hydroxybenzoic acid, hydantoin derivatives, propionate salts, and a variety of quaternary ammonium compounds. Particularly preferred preservatives of this invention are methyl paraben, propyl paraben, phenoxyethanol and benzyl alcohol. Preservatives will usually be employed in amounts ranging from about 0.1% to 2% by weight of the topical composition.

Still other optional ingredients/additives that may be used in the topical composition of this invention include chelators like EDTA, pH modifiers (e.g., NaOH), dioic acids (e.g., malonic acid, sebacic acid), antioxidants like vitamin E, retinoids, including retinoic acid, retinal, retinol and retinyl esters, conjugated linoleic acid, petroselinic acid and mixtures thereof, as well as any other conventional ingredients well known for wrinkle-reducing, anti-acne effects and reducing the impact of sebum.

Even other optional additives that may be employed in the topical composition of the present invention are skin lightening additives. Illustrative yet non-limiting examples of skin lightening additives that may be used in this invention are niacinamide, vitamin C and its derivatives, 12-hydroxystearic acid, resorcinols and their derivatives (including those esterified with, for example, ferulic acid, vanillic acid), extracts of kudzu, chamomile, and yarrow as well as any mixtures of the skin lightening sources.

Often preferred optional additives suitable for use in the topical composition of this invention include sensory modifying particles like microcrystalline cellulose, silica modified ethylene/methacrylate copolymer microspheres, talc modified ethylene/methacrylate copolymer microspheres, or mixtures thereof. Other examples of the types of particles suitable for use in this invention include those comprising polyolefins like polyethylene, polypropylene and/or polybutylene-based polymers, polyamides (like nylon fibers), or mixtures thereof. Still other preferred particles suitable for use in this invention include those comprising polyurethane, polystyrene, epoxy resins, urea resins, silicone resins, or mixtures thereof.

In a preferred embodiment, the particles used in this invention comprise polyethylenes, or are talc comprising particles or mixtures thereof. The former are often sold under the names Cerapure (made commercially available by Shamrock), Asensa (made commercially available by Honeywell) and Miperon (made commercially available by Mitsui Petrochemical Industries, Ltd.). Another preferred polyethylene-based particle is sold under the name CL-2080 (made commercially available by Kobo Industries). Other preferred particles suitable for use in this invention include nylons (e.g., nylon-12) sold under the name SP-10 which is made commercially available by Kobo Industries. Still other preferred particles suitable for use in this invention include those comprising copolymers of ethylene and methacrylate that contain silica or talc and sold under the names SPCAT-I2 and DSPCS-I2, respectively, both of which are also made commercially available by Kobo Industries. Other particles comprising polystyrenes and polymethyl methacrylate (sold, for example, under the names Ganzpearl GS-0605 and GME0380, respectively) and made available from Presperse are also often preferred.

Even other particles suitable for use in this invention include natural polymeric spheroids like those which comprise starch and those which comprise silk, the former, for example, made available from National Starch and Chemical and the latter, for example, made available by Engelhard Corporation. Still other natural polymeric particles suitable for use in this invention include those natural polymeric particles comprising cellulose such as Celluflow and Cellulo Beads, the former made commercially available by Chisso Corporation and the latter made available by Kobo Industries.

When used, such particles typically make up from about 0.001 to about 10%, and preferably, from about 0.01 to about 8%, and most preferably, from about 0.1 to about 6% by weight of the total weight of the topical composition, including all ranges subsumed therein.

Other preferred optional additives suitable for use with the HIPEs of this invention include moisturizing agents like hydroxypropyl tri(C₁-C₃ alkyl)ammonium salts. These salts may be obtained in a variety of synthetic procedures, most particularly by hydrolysis of chlorohydroxypropyl tri(C₁-C₃ alkyl)ammonium salts. A most preferred species is 1,2-dihydroxypropyltrimonium chloride, wherein the C₁-C₃ alkyl is a methyl group. Amounts of the salt may range from about 0.2 to about 30%, and preferably from about 0.5 to about 20%, optimally from about 1% to about 12% by weight of the topical composition, including all ranges subsumed therein.

Ordinarily the C₁-C₃ alkyl constituent on the quaternized ammonium group will be methyl, ethyl, n-propyl, isopropyl or hydroxyethyl and mixtures thereof. Particularly preferred is a trimethyl ammonium group known through INCI nomenclature as a "trimonium" group. Any anion can be used in the quat salt. The anion may be organic or inorganic with proviso that the material is cosmetically acceptable. Typical inorganic anions are halides, sulfates, phosphates, nitrates and borates. Most preferred are the halides, especially chloride. Organic anionic counter ions include methosulfate, toluoyl sulfate, acetate, citrate, tartrate, lactate, gluconate, and benzenesulfonate.

Still other preferred moisturizing agents which may be used, especially in conjunction with the aforementioned ammonium salts include substituted urea like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea. Where the term hydroypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from the National Starch & Chemical Division of ICI under the trademark Hydrovance.

Amounts of substituted urea that may be used in the topical composition of this invention range from about 0.01 to about 20%, and preferably, from about 0.5 to about 15%, and most preferably, from about 2 to about 10% based on total weight of the composition and including all ranges subsumed therein.

When ammonium salt and substituted urea are used, in a most especially preferred embodiment at least from about 0.01 to about 25%, and preferably, from about 0.2 to about 20%, and most preferably, from about 1 to about 15% humectant, like glycerine, is used, based on total weight of the topical composition and including all ranges subsumed therein. In yet another especially preferred embodiment, the topical composition of this invention is substantially free of silicone elastomer.

The topical composition of the present invention is intended for use primarily as a product for topical application to human skin, especially and at least as a product that may moisturize the skin. Thus, the inventors have discovered that the described HIPEs unexpectedly can be used as an excellent base in a topical composition to deliver excellent sensory benefits (e.g., silkiness) when the topical composition is, for example, substantially free of silicone elastomer. Other benefits from using the topical composition of this invention may include skin lightening, decreasing the effect of sebum on the skin and skin wrinkle reducing. In an especially preferred embodiment, the topical composition of the present invention has a pH from about 4.5 to about 7.5, including all ranges subsumed therein. Moreover, the topical composition of the present invention typically has a viscosity from about 4,000 to about 30,000, and preferably, from about 8,000 to about 25,000, and most preferably, from about 12,000 to about 23,000 cps (mPa·s) initially and after 24 hours at ambient temperature (measured with a Brookfield DV-1 Viscometer, with RV-S06 spindle, 25°C, 20 rpm).

When making the topical composition of the present invention, the desired GPS is generally added after other ingredients are mixed and at temperatures from about 20 to about 70°C and under atmospheric pressure.

The packaging for the composition of this invention can be a patch, bottle, tube, roll-ball applicator, propellant driven aerosol device, squeeze container or lidded jar.

The examples which follow are provided to illustrate and facilitate an understanding of the invention. The examples are not intended to limit the scope of the claims.

### EXAMPLES

### Example 1

The ingredients as listed in Table 1 below were used to prepare a typical HIPE (prior to gelation to form gel particle suspension) of the invention:

**Table 1**

| Material/Ingredient | Initial Phase for Preparation | % by wt. | Weight (Grams) | |
|---|---|---|---|---|
| Mineral Oil (Pioner 6501) | A1 | 5.00 | 12.50 | |
| KSG-310 (mixture of PEG-15/lauryl dimethicone cross-polymer and mineral oil*) | A2 | 5.00 | 12.50 | Oil Phase |
| Agarose | B1 | 1.80 | 4.50 | |
| Deionized water | B2 | 88.20 | 220.50 | Aqueous phase |

| | | | | |
|---|---|---|---|---|
| *KSG-310^{®} from Shin Etsu | | | | |

Using components of Table 1 above, the HIPE emulsion was prepared and cooled to form GPS as follows.

KSG-310 of phase A2 was mixed with mineral oil of phase A1 using standard mixing equipment and the two were heated above 75°C. In a separate beaker, agarose and water were combined (phase B) and heated with agitation to 75°C. When both aqueous phase B and oil phase A were at 75°C, phase B (aqueous phase) was slowly added to oil phase (A) under agitation. The components were mixed for about 10 minutes and then cooled under agitation. The resulting mixture comprises 90% by wt spherical agarose gel particles of below about 40 microns. It should be noted that no additional homogenization step was required and the particles are spherical.

Examples 2-17 as well as comparatives were prepared using the same procedure and the weight percentages of ingredients used are listed in the following table:

**Table 2**

| Examples | → | | | | | | | | | | | | | | | | Comparatives | | |
|---|---|---|---|---|---|---|---|---|---|---|---|---|---|---|---|---|---|---|---|
| Materials | 2 | 3 | 4 | 5 | 6 | 7 | 8 | 9 | 10 | 11 | 12 | 13 | 14 | 15 | 16 | 17 | A | B | C |
| KSG-310(PEG-15/Lauryl Dimethicone Cross Polymer & mineral oil) | 5 | 5 | 1 | | | | 1 | | | | | | | | | | | | |
| Cetyl PEG/PPG-10/1 Dimethicone (Abil EM 90) | | | | 1.5 | 0.19 | 0.15 | | | 0.15 | 0.15 | 0.15 | | | 0.5 | 0.5 | 0.5 | | 0.15 | 0.15 |
| Lauryl PEG-9 Polydimethylsiloxyethyl Dimethicone(KF6038) | | | | | | | | | | | | 0.15 | | | | | | | |
| PEG-10 Dimethicone (KF-6017) | | | | | | | | 0.15 | | | | | | | | | | | |
| Sorbitan monooleate (Span 80) | | | | | | | | | | | | | 0.15 | | | | | | |
| Mineral oil(Pionier 6501) | 5 | 5 | 9 | 8.5 | 9.81 | 7.55 | | | | | 6.15 | 7.54 | | | | 9.5 | 10 | | |
| Moneral oil(Lilac 100) | | | | | | | 9 | | 6.15 | 4.61 | | | 4.61 | | | | | 7.55 | |
| Silicone oil(DC200/50cts) | | | | | | | | 7.55 | 1.39 | 2.93 | 1.39 | | 2.93 | | | | | | 7.55 |
| Caprylic/capryl Triglyceride | | | | | | | | | | | | | | 9.5 | | | | | |
| Isopropyl Myristate | | | | | | | | | | | | | | | 9.5 | | | | |
| Agarose | 2.7 | 3.6 | 2.7 | 1.8 | 1.8 | 1.85 | 1.8 | 1.85 | 1.85 | 2.77 | 1.85 | 1.85 | 2.77 | 1.8 | 1.8 | 9 | 2.7 | 1.85 | 1.85 |
| Methylparaben | | | | | | | | | | 0.2 | 0.2 | | | | | | | | |
| DI water | 87.3 | 86.4 | 87.3 | 88.2 | 88.2 | 90.45 | 88.2 | 90.45 | 90.45 | 89.34 | 90.26 | 90.45 | 89.54 | 88.2 | 88.2 | 81 | 87.3 | 90.45 | 90.45 |
| | | | | | | | | | | | | | | | | | | | |
| Water phase, wt% | 90 | 90 | 90 | 90 | 90 | 92.3 | 90 | 92.3 | 92.3 | 92.31 | 92.31 | 92.3 | 92.31 | 90 | 90 | 90 | 90 | 92.3 | 92.3 |

Stable gel particle suspensions were formed with agarose gel particles of 1-50 microns for Examples 2-17. No stable GPS was formed in Comparatives A, B and C. Comparative A shows that surfactant is required to form GPS. Comparative B and C show certain combinations of surfactant and oils are not suitable in forming GPS with a high internal phase (up to 92.3% by wt) and at very low surfactant level (as low as 0.15%). More specifically, Abil em 90, which contains alkyl branches, tends to stabilize GPS with mineral oils as external phase more than silicone oils, which explains why Comparative B fails to form GPS. In addition, due to the specific structure of Abil em 90, it tends to stabilize GPS with heavier mineral oils (such as Pionier 6501) as external phase more than lighter ones (Lilac 100) as shown in comparative C.

Unexpectedly Abil em 90 can stabilize GPS with blends of silicone oil and lighter mineral oil from a ratio ranging 10:1 to 1:10. This can be explained by the fact that, in the oil blend, the lighter mineral oil imparts more affinity to the alkyl chains on Abil em 90 polymer and the silicone oil(e.g. DC200/50) improves the consistency of the oil blend.

### Example 18

In one aspect of the invention the invention relates to incorporation of a gel particle suspension using agarose gel into an aqueous skin care composition.

### Example 18A

Specifically, a composition (shown in Table 4) was made comprising:
1) 19.5% of the suspension of Example 14; and
2) 80.5% of base having a formulation composition as noted in Table 3.

**Table 3 Base formulation**

| Ingredient | Function | Wt./Wt. % |
|---|---|---|
| Distilled water | -- | ∼34% |
| Disodium EDTA | Preservative/chelator | 0.050 |
| Glycerine | Humectant | 3.0 |
| Preservatives | -- | 0.7 |
| Polyethylene (20) sorbitan monopalmitate | Surfactant | 2.0 |
| Silicone elastomer(DC 9041) | Particle and silicone volatile | 18.0 |
| Dimethicones 50cts | | 2.02 |
| Dimethicones 5cts | | 1.0 |
| DC 245 | Silicone volatile | 16.0 |
| Lilac 100 | Mineral oil/emollient | 0.3 |
| Koba MSP-825 (PMMA)¹ | Sensory modifier | 2.7 |
| Aristoflex AVC ex Clariant² | Thickener | 0.483 |

| | | |
|---|---|---|
| ¹ Methylmethacrylate cross-polymer ² Ammonium acryloyldimethyltaurate/vinylpyrolidone copolymer | | |

**Table 4 Final formulation of the topical product with Agarose Gel particles from Example 14**

| Ingredient | Function | Wt./Wt. % |
|---|---|---|
| Distilled water | -- | ∼34% |
| Disodium EDTA | Preservative/chelator | 0.050 |
| Glycerine | Humectant | 3.0 |
| Preservatives | -- | 0.7 |
| Polyethylene (20) sorbitan monopalmitate | Surfactant | 2.0 |
| Silicone elastomer(DC 9041) | Particle and silicone volatile | 18.0 |
| Dimethicones 50cts | | 2.59 |
| Dimethicones 5cts | | 1.0 |
| DC 245 | Silicone volatile | 16.0 |
| Lilac 100 | Mineral oil/emollient | 1.2 |
| Koba MSP-825 (PMMA) | Sensory modifier | 2.7 |
| Aristoflex AVC | Thickener | 0.483 |
| Agarose gel particles (from example 14 suspension) | Sensory modifier | 18.0 |
| Sorbitan monooleate (from example 14 suspension) | Surfactant | 0.03 |

The topical product base was mixed in a standard equipment. The Agarose gel particle suspension from Example 14 was then added to the base and mixed until uniform. The final composition is as shown in Table 4

### Example 18B

In comparison to Example 18A, another topical formulation was prepared as shown in Table 5, which is the same as Example 18A, except agarose gel was substituted with the same amount of water.

**Table 5. The topical product made substituting Agarose gel particle suspension with the same amount of water**

| Ingredient | Function | Wt./Wt. % |
|---|---|---|
| Distilled water | -- | ∼52% |
| Disodium EDTA | Preservative/chelator | 0.050 |
| Glycerine | Humectant | 3.0 |
| Preservatives | -- | 0.7 |
| Polyethylene (20) sorbitan monopalmitate | Surfactant | 2.0 |
| Silicone elastomer(DC 9041) | Particle and silicone volatile | 18.0 |
| Dimethicones 50cts | | 2.59 |
| Dimethicones 5cts | | 1.0 |
| DC 245 | Silicone volatile | 16.0 |
| Lilac 100 | Mineral oil/emollient | 1.2 |
| Koba MSP-825 (PMMA) | Sensory modifier | 2.7 |
| Aristoflex AVC | Thickener | 0.483 |
| Sorbitan monooleate | Surfactant | 0.03 |

The sensory attribute of the inventive composition, Example 18A, shown above relative to control (with no agarose), Example 18B, and Pond's® Fine Pore (a commercial product from Unilever with unique silkiness) was evaluated via a sensory panel which demonstrated that the inventive composition was perceived as being more silky than the composition containing no agarose, and in parity to the sensory feel of Pond's® Fine Pore.

## Claims

1. A highly concentrated, spherical, biopolymer gel particle suspension formed from a high internal phase emulsion (HIPE) intermediate wherein said suspension comprises:
a) an aqueous phase comprising 60 to 99% by wt. of said suspension of biopolymer gel particles;
wherein the ratio of biopolymer to water or polar solvent in the preparation of the aqueous phase is 0.01/99 to 5/85% by wt.;
wherein gel particles in the suspension are spherical; and wherein the average particle diameter of the particles in the suspension ranges from 1-50 micrometer; and
b) an oil phase comprising 1 to 40% by wt. of said suspension where the oil phase comprises a mixture of oils and surfactants used in the formation of a HIPE intermediate and wherein amounts of the oil and surfactant in HIPE intermediate are as follows:
(i) 0.1 to 30% by wt. of said HIPE intermediate comprising an oil or mixture of oils suspending said biopolymer particles of (a); and
(ii) 0.01 to 10% by wt. of said HIPE intermediate comprising surfactant or surfactants dissolved or dispersed in said oil or oil mixture of (b)(i),
wherein said surfactant comprises a nonionic surfactant having hydrophilic-lipophilic balance (HLB) less than 15;
wherein said final gel particle suspension is formed by first forming a HIPE intermediate product followed by cooling or gelling of such product to form the suspension.

2. An intermediate high internal phase emulsion (HIPE) which, prior to gellation, comprises:
1) an internal aqueous phase comprising:
a) 0.01 to 15% by wt. biopolymer relative to total amount of water and/or polar solvent;
b) 0 to 50% by wt. water soluble actives; and
c) 85 to 99% water and/or polar solvent
wherein internal phase has been mixed or heated until the appearance is clear before combining with an external phase defined by (2);
2) 0.1 to 40% by wt. of an external oil phase comprising:
a) 0.1 to 30% by wt. oil or oil blends;
b) 0.01 to 10% by wt. surfactant or surfactant mixtures
wherein said surfactant comprises a nonionic surfactant having HLB less than 15.

3. A suspension according to claim 1 wherein biopolymer gel particles comprise greater than 74 to 95% by wt. of suspension.

4. A suspension according to claim 1 or claim 3 wherein oil or oil mixture comprises 1 to 9% by wt. of suspension.

5. A suspension according to any one of claims 1, 3 or 4 wherein surfactant or surfactants comprise 0.1 to 2% by wt. of suspension.

6. A suspension according to any one of claims 1 or 3 to 5 wherein the nonionic surfactant has HLB less than 10.

7. A suspension according to any one of claims 1 or 3 to 6 wherein the particle diameter of the particle ranges from 1 to 50 micrometer.

8. A suspension according to any one of claims 1 or 3 to 7, wherein the biopolymer is selected from carrageenan furcellaren, pectin, alginate, agar, agarose, gellan, glucomannen, galactomannan, xanthan, modified cellulose, gelatin, whey protein and mixtures thereof.

9. A suspension according to any one of claims 1 or 3 to 8 wherein 0.01 to 15% biopolymer is used to control the elasticity or hardness of final biopolymer gel particles.

10. A suspension according to any one of claims 1 or 3 to 9 wherein polar solvent is selected from sorbitol, hydroxypropyl sorbitol, glycerine, ethoxylated glycerol, propolylated glycerol, polyalkylene glycols like polyethylene glycol and polypropylene glycol, diethylene glycol, dipropylene glycol, triethylene glycol, 2-ethoxyethanol, hexylene glycol, butylene glycol, hexamatriol.

11. A suspension according to any one of claims 1 or 3 to 10 wherein the water soluble actives are water soluble colorants and skin beneficial actives and are selected from the group consisting of glycolic acid, amino acids, glycerine, hydroxypropyl tri (C₁ to C₃ alkyl) ammonium salts and mixtures thereof.

12. A suspension according to any one of claims 1 or 3 to 11 wherein surfactant comprises linear, branched or cross-linked dimethicone polymers modified with polyether and/or alkyl chain; esters of sorbitan with fatty acids; cremaphor based surfactants; sucrose esters; monoglyceride based surfactants and mixtures thereof.

13. A suspension according to any one of claims 1 or 3 to 12 wherein the oil is selected from non-volatile mineral oil, organic oils, silicone oils and mixtures thereof.

14. A suspension according to claim 13 wherein the organic oil comprises triglyceride.

15. A suspension according to claim 13 or claim 14 wherein the organic oil comprises an ester of C₁-C₁₀ carbon of fatty acids with a C₈-C₂₄ chain length.

16. A suspension according to any one of claims 1 or 3 to 15 wherein combination of oil and surfactant excludes specific combinations consisting essentially of cetyl PEG/PPG-10/1 dimethicone and silicone oil when silicone oil is the only oil used in the preparation of HIPE intermediate.

17. A suspension according to any one of claims 1 or 3 to 16 wherein combination of oil and surfactant excludes cetyl PEG/PPG-10/1 dimethicone and light mineral oil when light mineral oil is the only oil used in the preparation of HIPE intermediate.

18. Topical composition which comprises the suspension of any one of claims 1 or 3 to 17 in a form selected from foams, liquids, lotions, creams, sera, gels, soap bars, cleansing products, toners and mixtures thereof.

19. A HIPE-gellation process for preparing a highly concentrated, spherical, biolpolymer gel particle suspension composition which composition comprises:
b) an aqueous phase comprising 60 to 99% by wt. of said suspension of spherical biolpolymer gel particles;
wherein the average diameter of the particles in the suspension is 1-50 micrometer;
wherein the biopolymer gel particles are formed from the aqueous internal phase of a high internal phase emulsion intermediate (HIPE) product whereby the aqueous internal phase comprises biopolymer and water or polar solvent and wherein the ratio of biopolymer to water or polar solvent is 0.01/99.99 to 15/85% by wt.; and
b) an oil phase comprising 1 to 40% by wt. of suspension where the oil phase comprises a mixture of oils and surfactants used in formation of said HIPE intermediate and wherein amounts of the oil and surfactants in said HIPE intermediate are as follows:
(i) 0.1 to 30% by wt. of said HIPE intermediate comprises an oil or mixture of oils suspending said biopolymer particles of (a); and
(ii) 0.01 to 10% by wt. of said HIPE intermediate product comprises surfactant or surfactants dissolved or dispersed in said oil or oil mixture of (b)(i)
wherein said surfactant comprises a nonionic surfactant having a hydrophilic-lipophiloc balance (HLB) less than 15,
wherein said process for forming the suspension composition comprises:
(A) forming an aqueous solution by dissolving biopolymer into water and/or polar solvent and heating dispersion to a temperature above the gellation temperature of the biopolymer;
(B) forming an oil solution by dissolving or dispersing surfactant(s) into the oil or mixture of oils, and heating said solution to a temperature above the temperature of the gellation temperature of the biopolymer;
(C) dispersing the biopolymer solution of (A) into the oil solution of (B) at a temperature above the gellation temperature of the biopolymer in order to form, as intermediate product, a water-in-oil high internal phase emulsion (HIPE) with biopolymer solution as internal phase in the form of small spherical droplets of 1 to 50µ suspended in oils found in the continuous oil phase; and
(D) cooling said HIPE containing biopolymer solution as internal phase to a temperature below the gellation temperature of biopolymer to form gel particle suspension composition defined by (a), (b)(i) and (b)(ii).

20. A process according to claim 19 wherein the HIPE is prepared in standard mixing equipment to a temperature 20% above the melting temperature of the biopolymer, within the range of 35°C to 100°C.

21. A process according to claim 19 or claim 20 wherein the HIPE is cooled to form the highly concentrated biopolymer gel particle suspension to a temperature below the gelling temperature of the biopolymer, within the range of 15°C to 45°C.

22. A process according to any one of claims 19 to 21 wherein oil or oil mixture comprises 1 to 9% by wt. of HIPE.

23. A process according to any one of claims 19 to 22 wherein the surfactant or surfactants comprise 0.1 to 2% by wt. of HIPE.

24. A process according to any one of claims 19 to 23 wherein the nonionic surfactant has HLB less than 10.

25. A process according to any one of claims 19 to 24 wherein the range of particle diameter of the particles is 5 to 40 micrometer.

26. A process according to any one of claims 19 to 25, wherein the biopolymer is selected from carrageenan furcellaren, pectin, alginate, agar, agarose, gellan, glucomannen, galactomannan, xanthan, modified cellulose, gelatin, whey protein and mixtures thereof.

27. A process according to any one of claims 19 to 26 wherein 0.01 to 15% of biopolymer is used to control the elasticity or hardness of final biopolymer gel particles.

28. A process according to any one of claims 19 to 27 wherein polar solvent is selected from the group consisting of sorbitol, hydroxypropyl sorbitol, glycerine, ethoxylated glycerol, propolylated glycerol, polyalkylene glycols like polyethylene glycol and polypropylene glycol, diethylene glycol, dipropylene glycol, triethylene glycol, 2-ethoxyethanol, hexylene glycol, butylene glycol, hexamatriol.

29. A process according to any one of claims 19 to 28 wherein water soluble active are water soluble colorants and skin beneficial agents selected from the group consisting of glycolic acid, amino acids, glycerine, hydroxypropyl tri (C₁ to C₃ alkyl) ammonium salts and mixtures.

30. A process according to any one of claims 19 to 29 wherein the surfactant is nonionic and comprises linear, branched or cross-linked dimethicone polymers modified with polyether and/or alkyl chain; esters of sorbitan with fatty acids; cremaphor based surfactants; sucrose esters; monoglyceride based surfactants and mixtures thereof.

31. A process according to any one of claims 19 to 30 wherein the oil is selected from non-volatile mineral oil, organic oils, silicone oils and mixtures thereof.

32. A process according to any one of claims 19 to 31 wherein combination of oil and surfactant excludes specific combination consisting essentially of cetyl PEG/PPG-10/1 dimethicone and silicone oil when silicone oil is the only oil used in the preparation of HIPE intermediate.

33. A process according to any one of claims 19 to 32 wherein combination of oil and surfactant excludes cetyl PEG/PPG-10/1 dimethicone and light mineral oil when light mineral oil is the only oil used in the preparation of HIPE intermediate.

34. A process according to any one of claims 19 to 33 for controlling elasticity or hardness of biopolymer gel particles comprising:
1) selecting proper combination of surfactant and optional oil during formation of the oil phase to obtain the desired hardness; and/or
2) controlling the concentration of biopolymer in biopolymer plus solvent solution.

## Patentansprüche

1. Suspension von hochkonzentrierten sphärischen Biopolymergelteilchen, die aus einem Zwischenprodukt in Form einer Emulsion mit hochkonzentrierter diskontinuierlicher Phase (HIPE) erzeugt ist, wobei die Suspension Folgendes aufweist:
a) eine wässrige Phase, die 60 bis 99 Gew.-% der Suspension von Biopolymergelteilchen ausmacht;
wobei das Verhältnis zwischen Biopolymer und Wasser oder polarem Lösungsmittel bei der Herstellung der wässrigen Phase 0,01/99 bis 5/85 Gew.-% beträgt;
wobei Gelteilchen in der Suspension sphärisch sind und wobei der mittlere Teilchendurchmesser der Teilchen in der Suspension im Bereich von 1 bis 50 µm liegt; und
b) eine Ölphase, die 1 bis 40 Gew.-% der Suspension ausmacht, wobei die Ölphase ein Gemisch von Ölen und oberflächenaktiven Mitteln aufweist, die bei der Herstellung des HIPE-Zwischenproduktes verwendet wurden, und wobei die Mengen an Öl und oberflächenaktivem Mittel im HIPE-Zwischenprodukt wie folgt sind:
(i) 0,1 bis 30 Gew.-% des HIPE-Zwischenproduktes weisen ein Öl oder ein Gemisch von Ölen auf, das die Biopolymerteilchen von (a) suspendiert; und
(ii) 0,01 bis 10 Gew.-% des HIPE-Zwischenproduktes weisen ein oberflächenaktives Mittel oder oberflächenaktive Mittel auf, das bzw. die in dem Öl oder Ölgemisch von (b)(i) gelöst oder dispergiert ist bzw. sind;
wobei das oberflächenaktive Mittel ein nichtionisches oberflächenaktives Mittel mit einem hydrophil-lipophilen Gleichgewicht (HLB) von weniger als 15 aufweist;
wobei die abschließende Gelteilchensuspension erzeugt wurde, indem zuerst ein HIPE-Zwischenprodukt erzeugt wird, gefolgt vom Abkühlen oder Bilden eines Gels dieses Produktes, um die Suspension zu erzeugen.

2. Zwischenprodukt in Form einer Emulsion mit hochkonzentrierter diskontinuierlicher Phase (HIPE),
das vor der Gelbildung Folgendes aufweist:
1) eine diskontinuierliche wässrige Phase, die Folgendes aufweist:
a) 0,01 bis 15 Gew.-% Biopolymer, auf die Gesamtmenge von Wasser und/oder polarem Lösungsmittel bezogen;
b) 0 bis 50 Gew.-% wasserlösliche wirksame Bestandteile; und
c) 85 bis 99 % Wasser und/oder polares Lösungsmittel;
wobei die diskontinuierliche Phase gemischt oder erwärmt worden ist, bis sie klar aussieht, bevor sie mit einer kontinuierlichen Phase kombiniert wird, wie sie unter (2) angegeben ist;
2) 0,1 bis 40 Gew.-% einer kontinuierlichen Ölphase, die Folgendes aufweist:
a) 0,1 bis 30 Gew.-% Öl oder Ölgemische;
b) 0,01 bis 10 Gew.-% oberflächenaktives Mittel oder Gemische von oberflächenaktiven Mitteln;
wobei das oberflächenaktive Mittel ein nichtionisches oberflächenaktives Mittel mit einem HLB-Wert von weniger als 15 aufweist.

3. Suspension nach Anspruch 1,
wobei die Biopolymergelteilchen mehr als 74 bis 95 Gew.-% der Suspension ausmachen.

4. Suspension nach Anspruch 1 oder 3,
wobei das Öl oder Ölgemisch 1 bis 9 Gew.-% der Suspension ausmacht.

5. Suspension nach einem der Ansprüche 1, 3 oder 4,
wobei das oberflächenaktive Mittel oder die oberflächenaktiven Mittel 0,01 bis 2 Gew.-% der Suspension ausmachen.

6. Suspension nach einem der Ansprüche 1 oder 3 bis 5,
wobei das nichtionische oberflächenaktive Mittel einen HLB-Wert von weniger als 10 aufweist.

7. Suspension nach einem der Ansprüche 1 oder 3 bis 6,
wobei der Teilchendurchmesser der Teilchen im Bereich von 1 bis 50 µm liegt.

8. Suspension nach einem der Ansprüche 1 oder 3 bis 7,
wobei das Biopolymer aus Carrageen, Furcellaren, Pektin, Alginat, Agar, Agarose, Gellan, Glucomannan, Galactomannan, Xanthan, modifizierter Cellulose, Gelatine, Molkeprotein und Gemischen davon ausgewählt ist.

9. Suspension nach einem der Ansprüche 1 oder 3 bis 8,
wobei 0,01 bis 15 % Biopolymer verwendet werden, um die Elastizität oder Härte von abschließenden Biopolymergelteilchen zu regeln.

10. Suspension nach einem der Ansprüche 1 oder 3 bis 9,
wobei das polare Lösungsmittel aus Sorbitol, Hydroxypropylsorbitol, Glycerin, ethoxyliertem Glycerol, propolyliertem Glycerol, Polyalkylenglycolen, wie Polyethylenglycol und Polypropylenglycol, Diethylenglycol, Dipropylenglycol, Triethylenglycol, 2-Ethoxyethanol, Hexylenglycol, Butylenglycol, Hexamatriol ausgewählt ist.

11. Suspension nach einem der Ansprüche 1 oder 3 bis 10,
wobei die wasserlöslichen wirksamen Bestandteile wasserlösliche färbende Mittel und für die Haut nützliche wirksame Mittel sind und aus der Gruppe ausgewählt sind, die aus Glycolsäure, Aminosäuren, Glycerin, Hydroxylpropyltri(C₁-C₃-alkyl)ammoniumsalzen und Gemischen davon besteht.

12. Suspension nach einem der Ansprüche 1 oder 3 bis 11,
wobei das oberflächenaktive Mittel lineare, verzweigte oder vernetzte Dimeticonpolymere, die mit Polyether und/oder einer Alkylkette modifiziert sind; Ester von Sorbitan mit Fettsäuren; auf Cremaphor basierende oberflächenaktive Mittel; Saccharoseester; auf Monoglycerid basierende oberflächenaktive Mittel und Gemische davon aufweist.

13. Suspension nach einem der Ansprüche 1 oder 3 bis 12,
wobei das Öl aus nicht-flüchtigem Mineralöl, organischen Ölen, Siliconölen und Gemischen davon ausgewählt ist.

14. Suspension nach Anspruch 13,
wobei das organische Öl Triglycerid aufweist.

15. Suspension nach Anspruch 13 oder 14,
wobei das organische Öl einen Ester mit C₁-C₁₀-Kohlenstoffatomen von Fettsäuren mit einer Kettenlänge von C₈-C₂₄ aufweist.

16. Suspension nach einem der Ansprüche 1 oder 3 bis 15,
wobei die Kombination von Öl und oberflächenaktivem Mittel bestimmte Kombinationen ausschließt, die im Wesentlichen aus Cetyl-PEG/PPG-10/1-Dimeticon und Siliconöl bestehen, wenn das Siliconöl das einzige Öl ist, das bei der Herstellung des HIPE-Zwischenproduktes verwendet wird.

17. Suspension nach einem der Ansprüche 1 oder 3 bis 16,
wobei die Kombination von Öl und oberflächenaktivem Mittel Cetyl-PEG/PPG-10/1-Dimeticon und leichtes Mineralöl ausschließt, wenn leichtes Mineralöl das einzige Öl darstellt, das bei der Herstellung des HIPE-Zwischenproduktes verwendet wird.

18. Topische Zusammensetzung,
die die Suspension nach einem der Ansprüche 1 oder 3 bis 17 aufweist, in einer Form, die aus Schäumen, Flüssigkeiten, Lotionen, Cremes, Sera, Gelen, Seifenstücken, Reinigungsprodukten, Tönungsmitteln und Gemischen davon ausgewählt ist.

19. HIPE-Gelbildungsverfahren zum Herstellen einer Zusammensetzung in Form einer Suspension von hochkonzentrierten, sphärischen Biopolymergelteilchen, wobei die Zusammensetzung Folgendes aufweist:
a) eine wässrige Phase, die 60 bis 99 Gew.-% der Suspension von sphärischen Biopolymergelteilchen ausmacht;
wobei der mittlere Durchmesser der Teilchen in der Suspension 1 bis 50 µm beträgt,
wobei die Biopolymergelteilchen aus der wässrigen diskontinuierlichen Phase eines Produktes in Form eines Zwischenproduktes aus einer Emulsion mit hochkonzentrierter diskontinuierlicher Phase (HIPE) erzeugt werden, wobei die wässrige diskontinuierliche Phase Biopolymer und Wasser oder polares Lösungsmittel aufweist und wobei das Verhältnis zwischen Biopolymer und Wasser oder polarem Lösungsmittel 0,01/99,99 bis 15/85 Gew.-% beträgt; und
b) eine Ölphase, die 1 bis 40 Gew. % der Suspension ausmacht, wobei die Ölphase ein Gemisch von Ölen und oberflächenaktiven Mitteln aufweist, die bei der Herstellung des HIPE-Zwischenproduktes verwendet wurden, und wobei die Mengen an Öl und oberflächenaktiven Mitteln im HIPE-Zwischenprodukt wie folgt sind:
(i) 0,1 bis 30 Gew. % des HIPE-Zwischenproduktes weisen ein Öl oder ein Gemisch von Ölen auf, das die Biopolymerteilchen von (a) suspendiert; und
(ii) 0,01 bis 10 Gew. % des HIPE-Zwischenproduktes weisen ein oberflächenaktives Mittel oder oberflächenaktive Mittel auf, das bzw. die in dem Öl oder Ölgemisch von (b)(i) gelöst oder dispergiert ist bzw. sind;
wobei das oberflächenaktive Mittel ein nichtionisches oberflächenaktives Mittel mit einem hydrophil-lipophilen Gleichgewicht (HLB) von weniger als 15 aufweist;
wobei das Verfahren zum Erzeugen der Zusammensetzung in Form einer Suspension Folgendes aufweist:
(A) Erzeugen einer wässrigen Lösung durch Auflösen von Biopolymer in Wasser und/oder polarem Lösungsmittel und Erwärmen der Dispersion auf eine Temperatur oberhalb der Gelbildungstemperatur des Biopolymers;
(B) Erzeugen einer Öllösung durch Auflösen oder Dispergieren von oberflächenaktivem Mittel (oberflächenaktiven Mitteln) im Öl oder im Gemisch von Ölen und Erwärmen der Lösung auf eine Temperatur oberhalb der Gelbildungstemperatur des Biopolymers;
(C) Dispergieren der Biopolymerlösung von (A) in der Öllösung von (B) bei einer Temperatur oberhalb der Gelbildungstemperatur des Biopolymers, um als Zwischenprodukt eine Wasser-in-Öl-Emulsion mit hochkonzentrierter diskontinuierlicher Phase (HIPE) mit der Biopolymerlösung als diskontinuierlicher Phase in Form von kleinen sphärischen Tröpfchen mit 1 bis 50 µm zu erzeugen, die in Ölen suspendiert sind, die in der kontinuierlichen Ölphase vorkommen; und
(D) Abkühlen der HIPE, die Biopolymerlösung als diskontinuierliche Phase enthält, auf eine Temperatur unterhalb der Gelbildungstemperatur des Biopolymers, um eine Zusammensetzung in Form einer Gelteilchensuspension, wie sie unter (a), (b)(i) und (b)(ii) angegeben ist, zu erzeugen.

20. Verfahren nach Anspruch 19,
wobei die HIPE in einer üblichen Mischvorrichtung bis zu einer Temperatur von 20 % oberhalb der Schmelztemperatur des Biopolymers in einem Bereich von 35 bis 100 °C hergestellt wird.

21. Verfahren nach Anspruch 19 oder 20,
wobei die HIPE auf eine Temperatur unterhalb der Gelbildungstemperatur des Biopolymers im Bereich von 15 bis 45 °C abgekühlt wird, um die Suspension mit hochkonzentrierten Biopolymergelteilchen zu erzeugen.

22. Verfahren nach einem der Ansprüche 19 bis 21,
wobei das Öl oder Ölgemisch 1 bis 9 Gew.-% der HIPE ausmacht.

23. Verfahren nach einem der Ansprüche 19 bis 22,
wobei das oberflächenaktive Mittel oder die oberflächenaktiven Mittel 0,1 bis 2 Gew.-% der HIPE ausmachen.

24. Verfahren nach einem der Ansprüche 19 bis 23,
wobei das nichtionische oberflächenaktive Mittel einen HLB-Wert von weniger als 10 aufweist.

25. Verfahren nach einem der Ansprüche 19 bis 24,
wobei der Bereich des Teilchendurchmessers der Teilchen 5 bis 40 µm beträgt.

26. Verfahren nach einem der Ansprüche 19 bis 25,
wobei das Biopolymer aus Carrageen, Furcellaren Pektin, Alginat, Agar, Agarose, Gellan, Glucomannan, Galactomannan, Xanthan, modifizierter Cellulose, Gelatine, Molkeprotein und Gemischen davon ausgewählt ist.

27. Verfahren nach einem der Ansprüche 19 bis 26,
wobei 0,01 bis 15 % Biopolymer verwendet werden, um die Elastizität oder Härte von abschließenden Biopolymergelteilchen zu regeln.

28. Verfahren nach einem der Ansprüche 19 bis 27,
wobei das polare Lösungsmittel aus der Gruppe ausgewählt ist, die aus Sorbitol, Hydroxypropylsorbitol, Glycerin, ethoxyliertem Glycerol, propolyliertem Glycerol, Polyalkylenglycolen, wie Polyethylenglycol und Polypropylenglycol, Diethylenglycol, Dipropylenglycol, Triethylenglycol, 2-Ethoxyethanol, Hexylenglycol, Butylenglycol, Hexamatriol besteht.

29. Verfahren nach einem der Ansprüche 19 bis 28,
wobei der wasserlösliche wirksame Bestandteil wasserlösliche färbende Mittel und für die Haut nützliche wirksame Mittel ist, die aus der Gruppe ausgewählt sind, die aus Glycolsäure, Aminosäuren, Glycerin, Hydroxylpropyltri(C₁-C₃-alkyl)ammoniumsalzen und Gemischen davon besteht.

30. Verfahren nach einem der Ansprüche 19 bis 29,
wobei das oberflächenaktive Mittel nichtionisch ist und lineare, verzweigte oder vernetzte Dimeticonpolymere, die mit Polyether und/oder einer Alkylkette modifiziert sind; Ester von Sorbitan mit Fettsäuren; auf Cremaphor basierende oberflächenaktive Mittel; Saccharoseester; auf Monoglycerid basierende oberflächenaktive Mittel und Gemische davon aufweist.

31. Verfahren nach einem der Ansprüche 19 bis 30,
wobei das Öl aus nicht-flüchtigem Mineralöl, organischen Ölen, Siliconölen und Gemischen davon ausgewählt ist.

32. Verfahren nach einem der Ansprüche 19 bis 31,
wobei die Kombination von Öl und oberflächenaktivem Mittel eine bestimmte Kombination ausschließt, die im Wesentlichen aus Cetyl-PEG/PPG-10/1-Dimeticon und Siliconöl bestehen, wenn das Siliconöl das einzige Öl ist, das bei der Herstellung des HIPE-Zwischenproduktes verwendet wird.

33. Verfahren nach einem der Ansprüche 19 bis 32,
wobei die Kombination von Öl und oberflächenaktivem Mittel Cetyl-PEG/PPG-10/1-Dimeticon und leichtes Mineralöl ausschließt, wenn leichtes Mineralöl das einzige Öl darstellt, das bei der Herstellung des HIPE-Zwischenproduktes verwendet wird.

34. Verfahren nach einem der Ansprüche 19 bis 33 zum Steuern der Elastizität oder Härte von Biopolymergelteilchen,
das Folgendes aufweist:
1) Auswählen einer geeigneten Kombination von oberflächenaktivem Mittel und wahlfreiem Öl während der Erzeugung der Ölphase, um die gewünschte Härte zu erreichen; und/oder
2) Steuern der Konzentration von Biopolymer in der Lösung von Biopolymer plus Lösungsmittel.

## Revendications

1. Suspension de particules de gel de biopolymère sphériques fortement concentrée formée à partir d'un intermédiaire d'émulsion à phase interne élevée (HIPE), dans laquelle ladite suspension comprend :
a) une phase aqueuse comprenant 60 à 99 % en poids de ladite suspension de particules de gel de biopolymère ;
dans laquelle le rapport entre le biopolymère et l'eau ou un solvant polaire dans la préparation de la phase aqueuse est de 0,01/99 à 5/85 % en poids ;
dans laquelle les particules de gel dans la suspension sont sphériques ; et dans laquelle le diamètre de particule moyen des particules dans la suspension va de 1 à 50 micromètres ; et
b) une phase huileuse comprenant 1 à 40 % en poids de ladite suspension où la phase huileuse comprend un mélange d'huiles et de tensioactifs utilisé dans la formation d'un intermédiaire HIPE et dans laquelle des quantités de l'huile et du tensioactif dans l'intermédiaire HIPE sont comme suit :
(i) 0,1 à 30 % en poids dudit intermédiaire HIPE comprenant une huile ou un mélange d'huiles mettant en suspension lesdites particules de biopolymère de (a) ; et
(ii) 0,01 à 10 % en poids dudit intermédiaire HIPE comprenant un ou des tensioactifs dissous ou dispersés dans ladite huile ou ledit mélange d'huiles de (b) (i),
dans laquelle ledit tensioactif comprend un tensioactif non ionique ayant un équilibre hydrophile-lipophile (HLB) inférieur à 15 ;
dans laquelle ladite suspension de particules de gel finale est formée en formant d'abord un produit intermédiaire HIPE, ceci étant suivi d'un refroidissement ou une gélification de ce produit pour former la suspension.

2. Intermédiaire d'émulsion à phase interne élevée (HIPE) qui, avant la gélification, comprend :
1) une phase aqueuse interne comprenant :
a) 0,01 à 15 % en poids de biopolymère par rapport à la quantité totale d'eau et/ou de solvant polaire ;
b) 0 à 50 % en poids d'actifs solubles dans l'eau ; et
c) 85 à 99 % d'eau et/ou de solvant polaire
dans laquelle la phase interne a été mélangée ou chauffée jusqu'à ce que l'apparence soit transparente avant combinaison avec une phase externe définie par (2) :
2) 0,1 à 40 % en poids d'une phase huileuse externe comprenant :
a) 0,1 à 30 % en poids d'huile ou de mélanges d'huiles ;
b) 0,01 à 10 % en poids de tensioactif ou de mélanges de tensioactifs
dans laquelle ledit tensioactif comprend un tensioactif non ionique ayant un HLB inférieur à 15.

3. Suspension selon la revendication 1, dans laquelle les particules de gel de biopolymère constituent plus de 74 à 95 % en poids de la suspension.

4. Suspension selon la revendication 1 ou la revendication 3, dans laquelle l'huile ou le mélange d'huiles constitue 1 à 9 % en poids de la suspension.

5. Suspension selon l'une quelconque des revendications 1, 3 ou 4, dans laquelle le ou les tensioactifs constituent 0,1 à 2 % en poids de la suspension.

6. Suspension selon l'une quelconque des revendications 1 ou 3 à 5, dans laquelle le tensioactif non ionique a un HLB inférieur à 10.

7. Suspension selon l'une quelconque des revendications 1 ou 3 à 6, dans laquelle le diamètre de particule de la particule va de 1 à 50 micromètres.

8. Suspension selon l'une quelconque des revendications 1 ou 3 à 7, dans laquelle le biopolymère est sélectionné parmi le carraghénane, le furcellarane, la pectine, l'alginate, l'agar-agar, l'agarose, la gellane, le glucomannane, le galactomannane, le xanthane, la cellulose modifiée, la gélatine, la protéine de lactosérum et leurs mélanges.

9. Suspension selon l'une quelconque des revendications 1 ou 3 à 8, dans laquelle 0,01 à 15 % de biopolymère sont utilisés pour réguler l'élasticité ou la dureté des particules de gel de biopolymère finales.

10. Suspension selon l'une quelconque des revendications 1 ou 3 à 9, dans laquelle le solvant polaire est sélectionné parmi le sorbitol, l'hydroxypropyl sorbitol, la glycérine, le glycérol éthoxylé, le glycérol propolylé, les poly(alkylène) glycols tels que le poly(éthylène) glycol et le poly(propylène) glycol, le diéthylène glycol, le dipropylène glycol, le triéthylène glycol, le 2-éthoxyéthanol, l'hexylène glycol, le butylène glycol, l'hexamatriol.

11. Suspension selon l'une quelconque des revendications 1 ou 3 à 10, dans laquelle les actifs solubles dans l'eau sont des colorants solubles dans l'eau et des agents bénéfiques pour la peau et sont sélectionnés dans le groupe consistant en l'acide glycolique, les acides aminés, la glycérine, les sels d'hydroxypropyl tri(alkyl en C₁ à C₃) ammonium et leurs mélanges.

12. Suspension selon l'une quelconque des revendications 1 ou 3 à 11, dans laquelle le tensioactif comprend des polymères de diméthicone linéaires, ramifiés ou réticulés modifiés par poly(éther) et/ou chaîne alkyle ; des esters de sorbitan avec des acides gras ; des tensioactifs à base de crémaphor ; des esters de saccharose ; des tensioactifs à base de monoglycéride et leurs mélanges.

13. Suspension selon l'une quelconque des revendications 1 ou 3 à 12, dans laquelle l'huile est sélectionnée parmi une huile minérale non volatile, des huiles organiques, des huiles de silicone et leurs mélanges.

14. Suspension selon la revendication 13, dans laquelle l'huile organique comprend un triglycéride.

15. Suspension selon la revendication 13 ou la revendication 14, dans laquelle l'huile organique comprend un ester de carbone en C₁ à C₁₀ d'acides gras avec une longueur de chaîne en C₈ à C₂₄.

16. Suspension selon l'une quelconque des revendications 1 ou 3 à 15, dans laquelle une combinaison d'huile et de tensioactif exclut des combinaisons spécifiques consistant essentiellement en la cétyl PEG/PPG-10/1 diméthicone et une huile de silicone lorsque l'huile de silicone est la seule huile utilisée dans la préparation d'intermédiaire HIPE.

17. Suspension selon l'une quelconque des revendications 1 ou 3 à 16, dans laquelle une combinaison d'huile et de tensioactif exclut la cétyl PEG/PPG-10/1 diméthicone et une huile minérale légère lorsque l'huile minérale légère est la seule huile utilisée dans la préparation de l'intermédiaire HIPE.

18. Composition topique qui comprend la suspension de l'une quelconque des revendications 1 ou 3 à 17 sous une forme sélectionnée parmi les mousses, les liquides, les lotions, les crèmes, les sérums, les gels, les pains de savon, les produits nettoyants, les toniques et leurs mélanges.

19. Procédé de gélification de HIPE pour la préparation d'une composition de suspension de particules de gel de biopolymère sphériques hautement concentrées, laquelle composition comprend :
b) une phase aqueuse comprenant 60 à 99 % en poids de ladite suspension de particules de gel de biopolymère sphériques ;
dans laquelle le diamètre moyen des particules dans la suspension est de 1 à 50 micromètres ;
dans laquelle les particules de gel de biopolymère sont formées à partir de la phase interne aqueuse d'un produit intermédiaire d'émulsion à phase interne élevée (HIPE), la phase interne aqueuse comprenant de ce fait un biopolymère et de l'eau et/ou un solvant polaire, et dans laquelle le rapport entre le biopolymère et l'eau ou le solvant polaire est de 0,01/99,99 à 15/85 % en poids ; et
b) une phase huileuse comprenant 1 à 40 % en poids de ladite suspension, où la phase huileuse comprend un mélange d'huiles et de tensioactifs utilisé dans la formation dudit intermédiaire HIPE et dans laquelle des quantités de l'huile et de tensioactifs dans ledit intermédiaire HIPE sont comme suit :
(i) 0,1 à 30 % en poids dudit intermédiaire HIPE comprennent une huile ou un mélange d'huiles mettant en suspension lesdites particules de biopolymère de (a) ; et
(ii) 0,01 à 10 % en poids dudit produit intermédiaire HIPE comprennent un ou des tensioactifs dissous ou dispersés dans ladite huile ou ledit mélange d'huiles de (b) (i),
dans laquelle ledit tensioactif comprend un tensioactif non ionique ayant un équilibre hydrophile-lipophile (HLB) inférieur à 15 ;
dans lequel ledit procédé de formation de la composition de suspension comprend :
(A) la formation d'une solution aqueuse en dissolvant un biopolymère dans l'eau et/ou un solvant polaire et en chauffant la dispersion à une température au-dessus de la température de gélification du biopolymère ;
(B) la formation d'une solution huileuse en dissolvant ou dispersant un ou des tensioactifs dans l'huile ou le mélange d'huiles et en chauffant ladite solution à une température au-dessus de la température de gélification du biopolymère ;
(C) la dispersion de la solution de biopolymère de (A) dans la solution huileuse de (B) à une température au-dessus de la température de gélification du biopolymère de façon à former, en tant que produit intermédiaire, une émulsion à phase interne élevée (HIPE) eau dans l'huile avec une solution de biopolymère en tant que phase interne sous forme de petites gouttelettes sphériques de 1 à 50 µm mises en suspension dans des huiles trouvées dans la phase huileuse continue ; et
(D) le refroidissement de ladite HIPE contenant la solution de biopolymère en tant que phase interne à une température en dessous de la température de gélification du biopolymère pour former une composition de suspension de particules de gel définie par (a), (b)(i) et (b)(ii).

20. Procédé selon la revendication 19, dans lequel la HIPE est préparée dans un équipement de mélange type à une température 20 % au-dessus de la température de fusion du biopolymère, dans la plage de 35 °C à 100 °C.

21. Procédé selon la revendication 19 ou la revendication 20, dans lequel la HIPE est refroidie pour former la suspension de particules de gel de biopolymère fortement concentrées à une température en dessous de la température de gélification du biopolymère, dans la plage de 15 °C à 45 °C.

22. Procédé selon l'une quelconque des revendications 19 à 21, dans lequel l'huile ou le mélange d'huiles constitue 1 à 9 % en poids de HIPE.

23. Procédé selon l'une quelconque des revendications 19 à 22, dans lequel le ou les tensioactifs constituent 0,1 à 2 % en poids de HIPE.

24. Procédé selon l'une quelconque des revendications 19 à 23, dans lequel le tensioactif non ionique a un HLB inférieur à 10.

25. Procédé selon l'une quelconque des revendications 19 à 24, dans lequel la plage du diamètre de particule des particules est de 5 à 40 micromètres.

26. Procédé selon l'une quelconque des revendications 19 à 25, dans lequel le biopolymère est sélectionné parmi le carraghénane, le furcellarane, la pectine, l'alginate, l'agar-agar, l'agarose, la gellane, le glucomannane, le galactomannane, le xanthane, la cellulose modifiée, la gélatine, la protéine de lactosérum et leurs mélanges.

27. Procédé selon l'une quelconque des revendications 19 à 26, dans lequel 0,01 à 15 % du biopolymère sont utilisés pour réguler l'élasticité ou la dureté des particules de gel de biopolymère finales.

28. Procédé selon l'une quelconque des revendications 19 à 27, dans lequel le solvant polaire est sélectionné dans le groupe consistant en le sorbitol, l'hydroxypropyl sorbitol, la glycérine, le glycérol éthoxylé, le glycérol propolylé, les poly(alkylène) glycols tels que le poly(éthylène) glycol et le poly(propylène) glycol, le diéthylène glycol, le dipropylène glycol, le triéthylène glycol, le 2-éthoxyéthanol, l'hexylène glycol, le butylène glycol, l'hexamatriol.

29. Procédé selon l'une quelconque des revendications 19 à 28, dans lequel les actifs solubles dans l'eau sont des colorants solubles dans l'eau et des agents bénéfiques pour la peau sélectionnés dans le groupe consistant en l'acide glycolique, les acides aminés, la glycérine, les sels d'hydroxypropyl tri(alkyle en C₁ à C₃)ammonium et leurs mélanges.

30. Procédé selon l'une quelconque des revendications 19 à 29, dans lequel le tensioactif est non ionique et comprend des polymères de diméthicone linéaires, ramifiés ou réticulés modifiés par poly(éther) et/ou chaîne alkyle ; des esters de sorbitan avec des acides gras ; des tensioactifs à base de crémaphor ; des esters de saccharose ; des tensioactifs à base de monoglycéride et leurs mélanges.

31. Procédé selon l'une quelconque des revendications 19 à 30, dans lequel l'huile est sélectionnée parmi une huile minérale non volatile, des huiles organiques, des huiles de silicone et leurs mélanges.

32. Procédé selon l'une quelconque des revendications 19 à 31, dans lequel une combinaison d'huile et de tensioactif exclut une combinaison spécifique consistant essentiellement en la cétyl PEG/PPG-10/1 diméthicone et l'huile de silicone lorsque l'huile de silicone est la seule huile utilisée dans la préparation de l'intermédiaire HIPE.

33. Procédé selon l'une quelconque des revendications 19 à 32, dans lequel une combinaison d'huile et de tensioactif exclut la cétyl PEG/PPG-10/1 diméthicone et une huile minérale légère lorsque l'huile minérale légère est la seule huile utilisée dans la préparation de l'intermédiaire HIPE.

34. Procédé selon l'une quelconque des revendications 19 à 33, pour la régulation de l'élasticité ou la dureté de particules de gel de biopolymère comprenant :
1) la sélection d'une combinaison correcte de tensioactif et d'une huile facultative pendant la formation de la phase huileuse pour obtenir la dureté souhaitée ; et/ou
2) la régulation de la concentration de biopolymère dans une solution biopolymère plus solvant.
